# EUROPÄISCHE PATENTSCHRIFT

(11) **EP 2 780 752 B1**
(45) Veröffentlichungstag und Bekanntmachung des Hinweises auf die Patenterteilung: **01.01.2020**
(21) Anmeldenummer: 12797776.7
(22) Anmeldetag: 15.11.2012
(51) Int. Cl.: G02B 21/00, A61B 3/13

(54) **JUSTIEREN EINER ANZEIGE FÜR ORIENTIERUNGSINFORMATION IN EINER VISUALISIERUNGSVORRICHTUNG**
ADJUSTING A DISPLAY FOR ORIENTATION INFORMATION IN A VISUALIZATION DEVICE
AJUSTEMENT D'UN AFFICHAGE POUR UNE INFORMATION D'ORIENTATION DANS UN DISPOSITIF DE VISUALISATION

(30) Priorität: 18.11.2011 DE 102011086666
(43) Veröffentlichungstag der Anmeldung: 24.09.2014
(73) Patentinhaber: Carl Zeiss Meditec AG, 07745 Jena (DE)
(72) Erfinder: WIRTH, Michael, 73434 Aalen (DE); THORNTON, Keith, 82178 Puchheim (DE)
(74) Vertreter: Gauss, Nikolai
(86) Internationale Anmeldenummer: PCT/EP2012/072748
(87) Internationale Veröffentlichungsnummer: WO 2013/072422

(56) Entgegenhaltungen:
- WO-A1-2004/034124
- DE-A1-102009 030 504
- US-A1- 2006 176 242
- US-A1- 2011 092 984
- US-B1- 6 483 948

## Beschreibung

Die Erfindung betrifft eine Visualisierungsvorrichtung mit einer Abbildungsoptik für das Erzeugen eines Beobachtungsbildes eines Objektbereichs, mit einer ein Display enthaltenden Anzeigeeinrichtung für das Visualisieren eines dem Beobachtungsbild des Objektbereichs überlagerten Bildes mit einer Orientierungsinformation, mit einer Bilderfassungseinrichtung für das Erfassen eines Bilds des Objektbereichs, und mit einem Rechnersystem, das aus einem mit der Bilderfassungseinrichtung erfassten Bild des Objektbereichs die Orientierungsinformation enthaltenden Bilddaten berechnet und an die Anzeigeeinrichtung übermittelt, wobei die Abbildungsoptik einen Binokulartubus für das Visualisieren des Objektsbereichs mit einem optischen Beobachtungsstrahlengang und einen in dem optischen Beobachtungsstrahlengang angeordneten Strahlteiler umfasst, der das Beobachtungsbild des Objektsbereichs der Bilderfassungseinrichtung zuführt, sowie einen in dem optischen Strahlengang angeordneten Strahlteiler aufweist, der das mit der Anzeigeeinrichtung angezeigte Bild dem Beobachtungsbild des Objektbereichs überlagert und dem Binokulartubus zuführt, und wobei das Rechnersystem bei der Berechnung der Bilddaten wenigstens einen von Fertigungs-, Einbau-, sowie Justagetoleranzen des Displays hervorgerufenen systematischen Fehler des mit der Anzeigeeinrichtung angezeigten Bildes gegenüber dem Beobachtungsbild korrigiert, indem es für das dem Beobachtungsbild überlagerte, angezeigte Bild wenigstens einen Korrekturparameter aus der Parametergruppe Vergrößerung, azimutale Verdrehung und Verschiebung einstellt, der aus einem Vergleich des Beobachtungsbildes eines Referenzobjekts mit einem dem Beobachtungsbild überlagerten und mit der Anzeigeneinrichtung (34) visualisierten Vergleichsobjekt ermittelt ist.

Unter einer Orientierungsinformation im Sinne der Erfindung werden insbesondere Hilfsgeometrien verstanden, die einem Beobachtungsbild in Operationsmikroskopen überlagert werden. Mit solchen Hilfsgeometrien ist es z. B. möglich, einem Operateur das Orientieren in einem Operationsgebiet zu erleichtern. Z.B. können mit solchen Hilfsgeometrien einem Operateur Gewebe- und Knochenstrukturen in einem Operationsgebiet präzise angezeigt werden. Orientierungsinformation im Sinne der Erfindung kann aber auch in Bildern bestehen, die z. B. mit diagnostischen Apparaten wie etwa MRT-Apparaten (Magnetic Resonance Tomography) oder MRI-Apparaten (Magnetic Resonance Imaging) an einem Patient präoperativ aufgenommen werden und die Strukturen in einem Operationsgebiet zeigen, die für eine Beobachtungsperson in dem Beobachtungsbild eines Objektbereichs nicht oder nur schwer zu erkennen sind.

Eine Visualisierungsvorrichtung der eingangs genannten Art ist aus der DE 10 2009 030 504 A1 bekannt. Dort ist eine als Operationsmikroskop ausgebildete Visualisierungsvorrichtung beschrieben, die eine Abbildungsoptik hat, die ein Beobachtungsbild einer Objektebene erzeugt. Dieses Operationsmikroskop ist ein Augenchirurgie-Mikroskopiesystem. Es enthält einen elektronischen Bildsensor, der mit einer Rechnereinheit für das Berechnen der Lage und Orientierung eines Patientenauges mittels Bildauswertung verbunden ist. Damit kann einer Beobachtungsperson Orientierungsinformation in Form einer berechneten Hilfsgeometrie angezeigt werden, die dem Beobachtungsbild eines Patientenauges korreliert überlagert ist.

Um einem Operateur ein genaues Orientieren in einem Operationsgebiet zu ermöglichen, ist es erforderlich, dass die angezeigte, überlagerte Orientierungsinformation zu dem Beobachtungsbild in einer solchen Visualisierungsvorrichtung exakt ausgerichtet ist.

In der WO 96/20421 A1 ist eine Visualisierungsvorrichtung für das Beobachten eines Objektbereichs beschrieben, die eine Rechnereinheit für die Korrektur von digital erzeugten Bildern ermöglicht, die dem Beobachtungsbild eines Objektbereichs überlagert sind. Die Visualisierungsvorrichtung ermöglicht auf diese Weise das Ausgleichen von Fehlern beim Erfassen der den digital erzeugten Bilder zugrundeliegenden Bilddaten und gewährleistet eine Anpassung von Bilddaten an die optischen Abbildungsfehler der Visualisierungsvorrichtung.

US2006176242 A1 offenbart eine Visualisierungsvorrichtung, in der ein Kamerabild mit einem realen Sichtfeld überlagert wird. Bei einer Kalibrierung durch den Benutzer kommt ein Kalibriergitter zum Einsatz.

Aufgabe der Erfindung ist es, eine Visualisierungsvorrichtung bereitzustellen, die das präzise Justieren von Orientierungsinformation ermöglicht, die einem Beobachtungsbild überlagert ist, und ein Verfahren anzugeben, mit dem ein systematischer Fehler von Orientierungsinformation in einer Visualisierungsvorrichtung exakt ermittelt werden kann, um diesen systematischen Fehler dann auszugleichen.

Diese Aufgabe wird durch eine Visualisierungsvorrichtung mit den Merkmalen des Anspruchs 1 und ein Verfahren mit den Merkmalen des Anspruchs 4 gelöst.

Bei der erfindungsgemäßen Visualisierungsvorrichtung enthält die Abbildungsoptik ein Vergrößerungssystem mit einer einstellbaren Vergrößerung und das Rechnersystem eine Eingabeschnittstelle für das Eingeben des wenigstens einen Korrekturparameters und für das Eingeben eines Anzeigeparameters zu unterschiedlichen Vergrößerungen sowie einen Datenspeicher, der den an der Eingabeschnittstelle zu unterschiedlichen Vergrößerungen eingegebenen Anzeigeparameter für das mit der Anzeigeeinrichtung angezeigte Bild speichert, wobei das Rechnersystem bei der Berechnung der für die Anzeige des Bildes (46) an die Anzeigeeinrichtung übermittelten Bilddaten die Vergrößerung des Vergrößerungssystems und die zu unterschiedlichen Vergrößerungen eingegebenen Korrekturparameter berücksichtigt.

Das erfindungsgemäße Verfahren sieht vor, dass der systematische Fehler für unterschiedliche Vergrößerungen der Visualisierungsvorrichtung ermittelt wird.

Die Erfinder haben erkannt, dass die Anzeige eines Bildes, das dem Beobachtungsbild eines Objektbereichs in einer Visualisierungsvorrichtung überlagert wird, häufig systematische Fehler aufweist, die sich nur mit sehr hohem Aufwand durch die Justage von optischen Baugruppen ausgleichen lassen. Diese systematischen Fehler können sich insbesondere bemerkbar machen, wenn dem Beobachtungsbild in einer Visualisierungsvorrichtung Orientierungsinformation überlagert werden soll, mit der die Position von Strukturen und Gebieten in einem Objektbereich angegeben wird, z. B. die Umrisse eines Tumors oder die Lage und Orientierung eines Patientenauges. Hier ist es nämlich wünschenswert, dass diese Orientierungsinformation allenfalls mit einem Fehler behaftet ist, der so gering ist, dass er die Genauigkeit von chirurgischen Eingriffen oder Diagnosen, die mit einer solchen Visualisierungsvorrichtung an einem Patient möglich sind, nicht beeinträchtigt, d. h. verringert.

Eine Idee der Erfindung ist es deshalb, in einer Visualisierungsvorrichtung eine Anzeigeeinrichtung, mit der ein berechnetes Bild visualisiert werden kann, das dem Bild eines Objektbereichs überlagert ist, rechnerisch zu justieren, indem die systematischen Fehler eines mit einem Display angezeigten Bildes, deren Ursache in den Fertigungs-, Einbau- sowie Justagetoleranzen des Displays liegt, bei der Berechnung der Bilddaten für die Anzeigeeinrichtung berücksichtigt werden. Damit lässt sich nämlich nicht nur Fertigungsaufwand für eine solche Visualisierungsvorrichtung verringern. Das Kompensieren von systematischen Fehlern eines in einer solchen Visualisierungsvorrichtung angezeigten Bildes mit einer Rechnereinheit eröffnet darüber hinaus auch eine Genauigkeit für die Anzeige von Orientierungsinformation, die mit den Methoden einer klassischen mechanischen Justage von Baugruppen gar nicht erreichbar ist.

In einer z. B. für das Gebiet der Neurochirurgie ausgelegten Visualisierungsvorrichtung kann diese Orientierungsinformation in den Umrissen eines Tumors in einem Operationsgebiet bestehen, oder in Gefäßen, die in einem Operationsgebiet verlaufen. In einer für das Gebiet der Ophthalmologie ausgebildeten Visualisierungsvorrichtung kann es sich bei der betreffenden Orientierungsinformation um den Kreis der Kapsularhexis, die Lage von torischen Linsen, die Hauptachsen eines Astigmatismus oder einen Nahtverlauf bei der Hornhautverpflanzung handeln.

Die Erfinder haben gesehen, dass das Kompensieren von systematischen Fehlern bei einer Anzeigeeinrichtung in einer Visualisierungsvorrichtung die Möglichkeit bietet, auch solche Fehler auszugleichen, die schon bei einem Erfassen von entsprechender Orientierungsinformation auftreten.

Die Eingabeschnittstelle des Rechnersystems ermöglicht ein Eingeben des wenigstens einen Korrekturparameters für das Justieren der Anzeigeeinrichtung in der Visualisierungsvorrichtung.

Das Rechnersystem kann mit dem Vergrößerungssystem verbunden sein und eine Rechnereinheit umfassen, die dann z.B. den wenigstens einen zur Einstellung verwendeten Korrekturparameter durch Auslesen und/oder Extrapolieren eines in dem Datenspeicher abgelegten Parameterkennfelds mit zu unterschiedlichen Vergrößerungen des Vergrößerungssystems hinterlegten Korrekturparametern bestimmt.

Für das Berechnen von Orientierungsinformation mittels Bildauswertung kann die Visualisierungsvorrichtung eine Bilderfassungseinrichtung enthalten, die einen Bildsensor für das Erfassen eines Bildes des Objektbereichs aufweist. Indem das mit dem Bildsensor erfasste Bild des Objektbereichs dem Rechnersystem zugeführt wird, ist es möglich, mittels Bildauswertung Bilddaten zu berechnen, die eine Orientierungsinformation enthalten. Das Rechnersystem kann hierzu z. B. die Bilddaten berechnen, indem das dem Bildsensor zugeführte Bild des Objektbereichs mit einer Vergleichsinformation korreliert wird. Diese Vergleichsinformation kann etwa in über das erfasste Bild des Objektbereichs gelegten Vergleichsobjekten bestehen, z.B. Vergleichsobjekten in Form von Ringfiltern, die einen inneren und einen äußeren Filterring haben, und Vergleichsobjekten in Form von Kreisringen, die mit wenigstens einer azimutalen Markierung versehen sind.

Eine Idee der Erfindung besteht nämlich insbesondere darin, auch systematische Fehler zu kompensieren, deren Ursache in den Fertigungstoleranzen eines Mikroskop-Hauptobjektivs liegt, insbesondere in den damit verbundenen Toleranzen für dessen Brennweite, in den mechanischen Fertigungs- und Justagetoleranzen eines Zoomsystems und in den mechanischen Fertigungs- und Justagetoleranzen einer Bilderfassungsoptik und eines Bildsensors in einer Bilderfassungseinrichtung. Bei der Berechnung eines dem Bild des Objektbereiches überlagerten angezeigten Bildes, das Orientierungsinformation enthält, die z. B. mittels Bildauswertung eines dem Bildsensor in der Bilderfassungseinrichtung zugeführten Bildes erzeugt ist, können diese Fehler dann ausgeglichen werden.

Die Visualisierungsvorrichtung kann insbesondere als ein Operationsmikroskop ausgebildet sein, das einen Binokulartubus für das Visualisieren des Objektbereichs mit einem optischen Beobachtungsstrahlengang aufweist und einen in dem optischen Beobachtungsstrahlengang angeordneten Strahlteiler enthält, der das Beobachtungsbild des Objektbereichs der Bilderfassungseinrichtung zuführt.

Die Anzeigeeinrichtung kann die Bildinformation dem Beobachtungsbild des Objektbereichs z.B. mit einem in dem optischen Strahlengang angeordneten Strahlteiler überlagern, der die Bildinformation dem Binokulartubus zuführt.

Um einen systematischen Fehler eines zu dem Beobachtungsbild eines Objektbereichs in einer Visualisierungsvorrichtung mit einer Anzeigeeinrichtung angezeigten Bildes zu ermitteln, wird ein Referenzobjekts in einem Objektbereich mit einer wenigstens die Lage und eine Länge sowie eine azimutale Orientierung des Referenzobjekts definierenden geometrischen Struktur bereitgestellt.

Darauf wird die geometrische Struktur eines Bildmusters in dem berechneten Bild wenigstens teilweise an die geometrische Struktur des in dem Beobachtungsbildes angezeigten Referenzobjekts durch Einstellen wenigstens eines Korrekturparameters für das mit der Anzeigeeinrichtung angezeigte Bild angepasst, der zu der Parametergruppe Vergrößerung, azimutale Verdrehung und Verschiebung gehört. Dann wird der systematische Fehler als derjenige wenigstens eine eingestellte Korrekturparameter bestimmt, bei dem wenigstens eine geometrische Struktur des Bildes an eine dieser Struktur entsprechende geometrische Struktur des angezeigten Beobachtungsbildes des Referenzobjekts wenigstens teilweise angepasst ist.

Die Anzeigeeinrichtung weist hierzu eine Abbildungsoptik für das Erzeugen eines Beobachtungsbildes eines Objektbereichs auf und hat eine Anzeigeeinrichtung mit einem Display für das Visualisieren eines dem Beobachtungsbild des Objektbereichs überlagerten Bildes mit einer Orientierungsinformation, die eine Bilderfassungseinrichtung für das Erfassen eines Bilds des Objektbereichs und ein Rechnersystem umfasst, das aus einem mit der Bilderfassungseinrichtung erfassten Bild des Objektbereichs die Orientierungsinformation enthaltenden Bilddaten berechnet und an die Anzeigeeinrichtung übermittelt.

Für das Visualisieren des Objektsbereichs mit einem optischen Beobachtungsstrahlengang hat die Abbildungsoptik einen Binokulartubus und enthält einen in dem optischen Beobachtungsstrahlengang angeordneten Strahlteiler, der das Beobachtungsbild des Objektsbereichs der Bilderfassungseinrichtung zuführt. Darüber hinaus hat die Abbildungsoptik einen in dem optischen Strahlengang angeordneten weiteren Strahlteiler, der das mit der Anzeigeeinrichtung angezeigte Bild dem Beobachtungsbild des Objektbereichs überlagert und dem Binokulartubus zuführt.

Das Beobachtungsbild des in dem Objektbereich bereitgestellten Referenzobjekts wird auf diese Weise mit dem an der Anzeigeeinrichtung berechneten Bild verglichen. Anschließend wird durch Einstellen wenigstens eines Korrekturparameters für das mit der Anzeigeeinrichtung angezeigte Bild aus der Parametergruppe Vergrößerung, azimutale Verdrehung und Verschiebung eine geometrische Struktur in dem berechneten Bild wenigstens teilweise mit der geometrischen Struktur des Referenzobjekts zur Deckung gebracht. Der gesuchte systematische Fehler wird dann zu demjenigen wenigstens einen eingestellten Korrekturparameter ermittelt, bei dem die geometrische Struktur des Referenzobjekts und die geometrische Struktur des berechneten Bildes wenigstens teilweise zur Deckung gebracht ist.

Dieser systematische Fehler wird vorteilhafterweise für unterschiedliche Vergrößerungen der Visualisierungsvorrichtung ermittelt. Um das mit einer Anzeigeeinrichtung angezeigte, berechnete Bild, das dem Beobachtungsbild überlagert ist, an das Beobachtungsbild anzupassen, wird ein ermittelter systematischer Fehler des dem Beobachtungsbild des Objektbereichs überlagerten mit der Anzeigeeinrichtung angezeigten berechneten Bildes kompensiert. Das Kompensieren erfolgt durch Einstellen wenigstens eines Korrekturparameters für das mit der Anzeigeeinrichtung angezeigte Bild aus der Parametergruppe Vergrößerung, azimutale Verdrehung und Verschiebung. D. h., es wird die Vergrößerung, azimutale Verdrehung oder Position des angezeigten Bildes als Ganzes oder eines Teils des angezeigten Bildes, insbesondere einer Struktur in dem angezeigten Bild korrigiert.

Entsprechende systematische Fehler können bei einer Visualisierungsvorrichtung nach der Erfindung sowohl bei deren Herstellung als auch bei nachträglichen Wartungsarbeiten vergleichsweise einfach ausgeglichen werden.

Die Rechnereinheit der Visualisierungsvorrichtung enthält hierzu ein Computerprogramm. Mit dem Computerprogramm werden aus einem Bild eines Objektbereichs, das mit einem Bildsensor aufgenommen wird, Bilddaten für eine Anzeigeeinrichtung berechnet. Das Computerprogramm berücksichtigt bei der Berechnung der an die Anzeigeeinrichtung übermittelten Bilddaten wenigstens einen in dem Rechnersystem gespeicherten Korrekturparameter aus der Parametergruppe Vergrößerung, azimutale Verdrehung und Verschiebung. Das Computerprogramm ist vorteilhafterweise so ausgelegt, dass es bei der Berechnung der für die Anzeige des Bildes an die Anzeigeeinrichtung übermittelten Bilddaten insbesondere die Vergrößerung des Beobachtungsbildes und zu unterschiedlichen Vergrößerungen des Beobachtungsbildes ermittelte Korrekturparameter berücksichtigt. Das Computerprogramm kann derart programmiert werden, dass es das dem Bildsensor zugeführte Bild des Objektbereichs mit einer Vergleichsinformation korreliert, insbesondere mit einer Vergleichsinformation in Form von über das erfasste Bild des Objektbereichs gelegten Vergleichsobjekten in Form von Ringfiltern, die einen inneren und einen äußeren Filterring haben, in denen das Vorzeichen der Filterfunktion bevorzugt verschieden ist, und in Form von Kreisringen mit wenigstens einer in einem Kreisring angeordneten Markierung.

Im Folgenden wird die Erfindung anhand der in der Zeichnung in schematischer Weise dargestellten Ausführungsbeispiele näher erläutert.

Es zeigen:
- Fig. 1: eine Visualisierungsvorrichtung mit einem Rechnersystem und einer Anzeigeeinrichtung, die ein Display enthält;
- Fig. 2a: und Fig. 2b das Display der Anzeigeeinrichtung mit einem angezeigten Bild;
- Fig. 3: ein Referenzobjekt mit einem Muster für das Justieren der Anzeige von Bildinformation, die einem Beobachtungsbild überlagert ist;
- Fig.4: eine an dem Display der Anzeigeeinrichtung angezeigte Bildinformation, die auf aus einem Bild des Musters in der Rechnereinheit berechneten Bilddaten basiert; und
- Fig. 5: ein das Justieren der Anzeige von Orientierungsinformation erläuterndes Flussdiagramm.

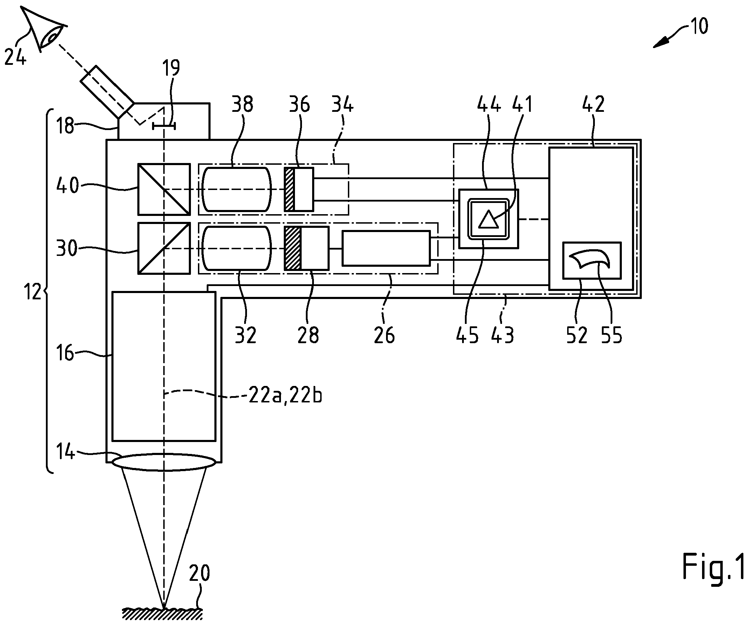

Die Visualisierungsvorrichtung in Fig. 1 ist ein Ophthalmo-Operationsmikroskop 10. Das Operationsmikroskop 10 hat eine Abbildungsoptik 12 mit einem Mikroskop-Hauptobjektiv 14, einem Zoomsystem 16 sowie einem Binokulartubus 18. Das Operationsmikroskop 10 ermöglicht das Beobachten eines Objektbereichs 20 mit einem binokularen Beobachtungsstrahlengang 22a, 22b. In dem Binokulartubus 18 wird den Augen 24 einer Beobachtungsperson ein Beobachtungsbild des Objektbereichs 20 zur Anzeige gebracht. Das Zoomsystem 16 ist verstellbar. Durch Verstellen des Zoomsystems kann die Vergrößerung Γ eines in dem Binokulartubus 18 erzeugten Zwischenbildes 19 in dem Bereich 0,4 ≤ Γ ≤ 2,4 variiert werden.

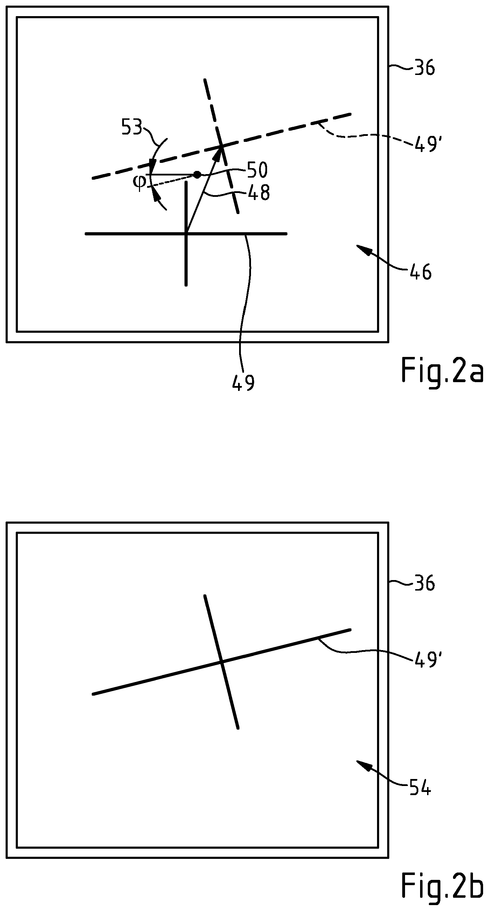

Das Operationsmikroskop 10 enthält eine Bilderfassungseinrichtung 26. In der Bilderfassungseinrichtung 26 gibt es einen Bildsensor 28. Dem Bildsensor 28 wird über einen in einem Beobachtungsstrahlengang 22a angeordneten Strahlteiler 30 und eine Bilderfassungsoptik 32 ein Bild des Objektbereichs 20 zugeführt. Um den Augen 24 einer Beobachtungsperson ein in Fig. 2 gezeigtes Bild 46 mit Orientierungsinformation 49 zu visualisieren, die dem Beobachtungsbild des Objektbereichs 20 in dem Binokulartubus 18 überlagert ist, gibt es in dem Operationsmikroskop 10 eine Anzeigeeinrichtung 34. Die Anzeigeeinrichtung 34 enthält ein Display 36 und hat eine Abbildungsoptik 38. Mittels der Abbildungsoptik 38 wird an dem Display 36 das zur Anzeige gebrachte Bild 46 mit Orientierungsinformation 49 zu einem in dem Beobachtungsstrahlengang 22b angeordneten Strahlteiler 40 geführt. Mit dem Strahlteiler 40 wird das an dem Display 36 angezeigte Bild 46 dem Beobachtungsstrahlengang 22b überlagert und in den Binokulartubus 18 gelenkt.

In dem Operationsmikroskop 10 gibt es ein Rechnersystem 43 mit einer Rechnereinheit 42 und einer Eingabeschnittstelle 44. Die Rechnereinheit 42 ist mit der Bilderfassungseinrichtung 26 verbunden und an die Anzeigeeinrichtung 34 angeschlossen. Das Operationsmikroskop 10 enthält eine Eingabeschnittstelle 44 für die Rechnereinheit 42. Die Rechnereinheit 42 erhält von der Bilderfassungseinrichtung 26 ein mit dem Bildsensor 28 erfasstes Bild des Objektbereichs 20. In dem Rechnersystem 43 werden aus dem Bild des Objektbereichs 20 mit der Rechnereinheit 42 Bilddaten berechnet und an die Anzeigeeinrichtung 34 für die Anzeige eines Bildes 46 mit einer Orientierungsinformation übermittelt.

An der Eingabeschnittstelle 44 kann eine Beobachtungsperson für das mit der Anzeigeeinrichtung 34 angezeigte Bild 46 Korrekturparameter einstellen. Die Eingabeschnittstelle 44 hat hierfür einen berührungssensitiven Monitor 45, auf dem die Beobachtungsperson mit den Fingern einer Hand die Abbildungsparameter für eine an dem Monitor 45 angezeigte geometrische Struktur 41 einstellen kann. Alternativ hierzu kann die Eingabeschnittstelle aber auch als Tastatur ausgebildet sein oder eine Sprachsteuereinrichtung aufweisen. Darüber hinaus ist es z. B. möglich, die Eingabeschnittstelle als Schnittstelle für den Anschluss einer weiteren Rechnereinheit oder eines Rechnernetzwerks auszubilden. Die an der Eingabeschnittstelle 44 eingegebenen Korrekturparameter werden aufgrund eines dem Rechnersystem 43 über die Eingabeschnittstelle 44 zuführbaren Steuerbefehls bei der Berechnung der an die Anzeigeeinrichtung 34 übermittelten Bilddaten berücksichtigt. Als Korrekturparameter kann an der Eingabeschnittstelle 44 die Position und die azimutale Orientierung sowie die Vergrößerung für das mit der Anzeigeeinrichtung 34 angezeigten Bildes 46 eingegeben und damit eingestellt werden.

Das Rechnersystem 43 des Operationsmikroskops 10 hat einen Datenspeicher 52. In dem Datenspeicher 52 können an der Eingabeschnittstelle 44 zu unterschiedlichen Vergrößerungen des Zoomsystems 16 eingegebene Korrekturparameter in Form eines Parameterkennfelds 55 abgelegt werden. Das Parameterkennfeld 55 kann z. B. eine Matrix sein, in deren Spalten die für eine bestimmte Vergrößerung ermittelten Korrekturparameter über die Position und azimutale Orientierung sowie die Vergrößerung aufgeführt sind.

Die Fig. 2a zeigt das Display 36 mit einem Bild 46, das an dem Display 36 angezeigt wird. Die Orientierungsinformation 49 in dem Bild 46 ist eine Hilfsgeometrie für das Anzeigen der Position und der azimutalen Orientierung einer torischen Intraokularlinse. Der in dem Datenspeicher 52 gespeicherte Korrekturparameter über die Position entspricht mathematisch einer in der Fig. 2a mit dem Pfeil 48 angedeuteten zweidimensionalen Verschiebeoperation, die auf die Hilfsgeometrie 49 in dem mit dem Display 36 angezeigten Bild 46 angewendet wird. Der Korrekturparameter über die azimutale Orientierung für das Bild 46 an dem Display 36 bedeutet mathematisch das azimutale Verdrehen der Hilfsgeometrie 49 an dem Display 36 um das Zentrum 50 mit dem Winkel ϕ in der Richtung des Pfeils 53 entsprechend einer auf die Hilfsgeometrie 49 angewendeten Drehoperation. Der Korrekturparameter über die Vergrößerung für das mit der Anzeigeeinrichtung 34 angezeigte Bild 46 hat die mathematische Bedeutung der Skalierung der an dem Display 36 in dem Bild 46 angezeigten Hilfsgeometrie 49. Die Hilfsgeometrie 49 in dem Bild 46 wird durch Verschieben, azimutales Verdrehen und Vergrößern in die Hilfsgeometrie 49' des in der Fig. 2b gezeigten mit dem Display 34 erzeugten, korrigierten Bildes 54 überführt.

Es sei bemerkt, dass das Einstellen eines Korrekturparameters für das mit der Anzeigeeinrichtung 34 angezeigte Bild in einer alternativen, erfindungsgemäßen Ausführungsform des Operationsmikroskops 10 auch das Verschieben, azimutale Verdrehen oder Vergrößern eines mit dem Display 36 erzeugten Bildes 46 als Ganzes bewirken kann.

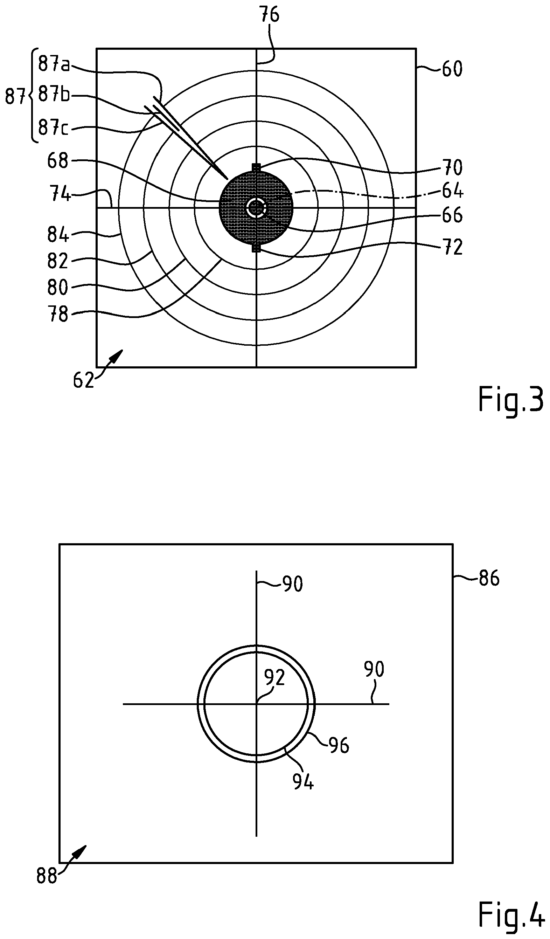

Die Fig. 3 zeigt ein Referenzobjekt 60 für das Justieren der Anzeige von mit der Anzeigeeinrichtung 34 in dem Operationsmikroskop 10 angezeigter Orientierungsinformation. Das Referenzobjekt 60 ist ein Glaskörper, auf dem ein als Maßstab ausgebildetes Muster 62 angebracht ist. Das Muster 62 hat die Struktur einer Zielscheibe mit einem Zentrum 64. Das Muster 62 umfasst eine zu dem Zentrum 64 konzentrisch angeordnete Kreisscheibe 66 mit dem Durchmesser 2,5mm, die sich in einer geometrischen Struktur in Form eines hierzu konzentrisch angeordneten Kreisrings 68 befindet. Der innere Durchmesser des Kreisrings 68 beträgt 5mm, der äußere Durchmesser 15mm. An dem äußeren Umfang des Kreisrings 68 gibt es zwei balkenförmige Markierungen 70, 72, die einander gegenüberliegen. Das Muster 62 enthält zwei zueinander senkrecht angeordnete Linien 74, 76, die einen Schnittpunkt haben, der mit dem Zentrum 64 zusammenfällt. Die beiden Markierungen 70, 72 befinden sich auf der Linie 76. Das Muster 62 hat außerdem vier zueinander konzentrische Kreislinien 78, 80, 82 und 84, die konzentrisch um den Kreisring 68 herum angeordnet sind. Der Radius der konzentrischen Kreislinie beträgt 25mm, 35mm, 45mm und 55mm. Das Muster 62 enthält zusätzlich eine Azimutwinkelmarkierung 87 mit drei, in dem Winkelabstand von 1° aufeinanderfolgend angeordneten Winkelstrichen 87a, 87b, 87c. Die Linien, Markierungen und Winkelstriche des Musters 62 auf dem Glaskörper 60 sind mit einer Genauigkeit von ±20 µm gefertigt. Mit den Linien 74, 76 und den Kreislinien 78, 80, 82, 84 hat das Muster 62 geometrische Strukturen, mit denen die Lage des Musters 62, eine Länge, d.h. die Größe des Musters 62, sowie die azimutale Orientierung des Musters 62 definiert ist. Damit wird erreicht, dass für eine optische Abbildung des Musters 62 anhand der geometrischen Strukturen des Musters 62 bestimmt werden kann, ob das Muster 62 bei der Abbildung gedreht und/oder verschoben und/oder vergrößert bzw. verkleinert wird.

Das Rechnersystem 43 in dem Operationsmikroskop 10 in Fig. 1 enthält ein Computerprogramm mit einer Bildverarbeitungsroutine, die unter Verwendung der Bilddaten eines dem Rechnersystem 43 von dem Bildsensor 28 zugeführten Bildes mit dem Muster 62 Bilddaten für die Anzeige eines Bildes 86 berechnet, das Orientierungsinformation in Form eines Bildmusters 88 enthält und das in der Fig. 4 gezeigt ist. Das Bildmuster 88 ist eine Hilfsgeometrie für das Justieren der Anzeige des Bildes 86 an dem Display 36.

Das in dem Rechnersystem aus dem Bild eines Objektbereichs 20 mit dem Referenzobjekt 60 berechnete Bild 86 mit dem Bildmuster 88 hat mehrere geometrische Strukturen. Das Bildmuster 88 enthält als eine erste geometrische Struktur ein Fadenkreuz 90 mit einem Zentrum 92. Als eine zweite geometrische Struktur hat das Bildmuster 88 zwei zu dem Zentrum 92 konzentrische Kreisringe 94, 96. Für eine Beobachtungsperson ist das in dem Binokulartubus 18 der Visualisierungsvorrichtung 10 wahrnehmbare Bildmuster 88, das dem Bild des Objektbereichs 20 überlagert ist, eine Orientierungsinformation über die Lage, die azimutale Orientierung und die Größe der in Form des Musters 62 auf dem in dem Objektbereich 20 angeordneten Referenzobjekt 60.

Für das Berechnen des Bildes 86 korreliert die Rechnereinheit 42 z.B. zunächst das mittels des Bildsensors 28 erfasste Beobachtungsbild des Objektbereichs 20 mit ringförmigen Vergleichsobjekten unterschiedlicher Größe. Dies ist detailliert auf S. 3, Z. 12 bis S. 4, Z. 14 und S. 5, Z. 9 bis S. 9, Z. 15 der internationalen Patentanmeldung mit Az. PCT/EP2008/068104 und auch in der internationalen Patentanmeldung mit Az. PCT/EP2008/068103 beschrieben, auf die hiermit vollumfänglich Bezug genommen wird. Damit wird das Zentrum 64 des Musters 62 und der äußere Radius r₁ des Kreisrings 68 bestimmt. Wenn das Zentrum 64 des Musters 62 und der äußere Radius des Kreisrings 68 ermittelt ist, wird das mittels des Bildsensors 28 erfasste Bild des Objektbereichs 20 in Polarkoordinaten transformiert und dann die azimutale Lage der Markierungen 70, 72 durch Korrelation mit zwei als Azimutwinkelmarkierungen wirkenden Flächenelementen korreliert, deren Winkelabstand W in Polarkoordinaten W = 180° beträgt, die sich über einen Radiusbereich r₁ ≤ r ≤ r₂ erstrecken, wobei r₁ dem äußere Radius des Kreisrings 68 entspricht und r₂ := 1,2 r₁ dem Radius r₂ eines um den Kreisring 68 gelegten weiteren Kreisrings entspricht, der zu dem Kreisring 68 konzentrisch ist.

Die Korrelation erfolgt durch Berechnen einer Korrelationsfunktion, z.B. unter Variation des Ortes, so dass die Korrelationsfunktion eine Funktion der Ortsvariablen ist. Dabei werden die Werte der Bildpunkte des Bildes mit den Werten der Bildpunkte des Vergleichsobjekts verrechnet, während das Vergleichsobjekt über das Bild bewegt wird. Der Wert der Korrelationsfunktion ist ein Maß für die Übereinstimmung von Bild und Vergleichsobjekt. Bei der maximalen Übereinstimmung von Bild und Vergleichsobjekt, d. h. wenn das charakteristische Merkmal des Vergleichsobjekts und das gesuchte charakteristische Merkmal im Bild übereinander liegen, ist der Wert der Korrelationsfunktion z. B. maximal.

Eine an dem Display 36 der Anzeigeeinrichtung 34 zur Anzeige gebrachte Orientierungsinformation unterliegt systematischen Fehlern. Die Ursache dieser Fehler sind insbesondere die Fertigungstoleranzen für die Brennweite des Mikroskop-Hauptobjektivs 14, Fertigungs- und Justagetoleranzen für das Zoomsystem 16 sowie Fertigungs- und Justagetoleranzen für die Bilderfassungsoptik 32 und den Bildsensor 28 in der Bilderfassungseinrichtung 26 in dem Operationsmikroskop 10. Auch die Fertigungs- und Justagetoleranzen für die Abbildungsoptik 38 und das Display 36 in der Anzeigeeinrichtung 34 und Fertigungs- und Justagetoleranzen für die Strahlteiler 30, 40 in dem Operationsmikroskop 10 tragen zu diesen systematischen Bildinformationsfehlern bei.

Die Anzeige von Orientierungsinformation kann in dem Operationsmikroskop 10 durch eine Datenkorrektur justiert werden, indem die vorgenannten systematischen Bildinformationsfehler für unterschiedliche, mögliche Vergrößerungen bei dem Operationsmikroskop 10 ermittelt und dann kompensiert werden.

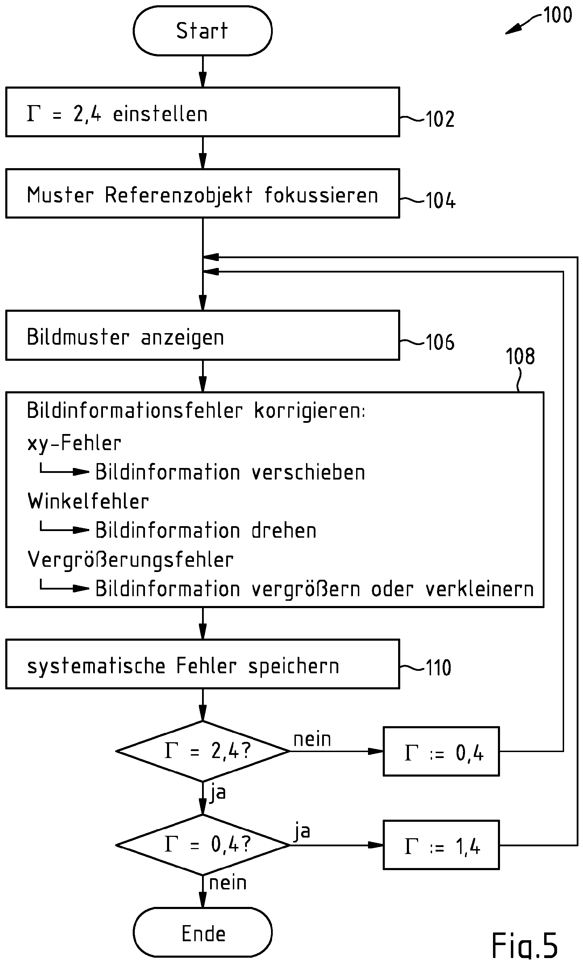

Die Fig. 5 zeigt ein Flussdiagramm 100, das den Ablauf einer Justage der Anzeige eines Bildes mit Orientierungsinformation erläutert, das mit dem Display 36 in der Anzeigeeinrichtung 34 in dem Operationsmikroskop 10 angezeigt wird. Hierfür wird das Referenzobjekt 60 in dem Objektbereich 20 des Operationsmikroskops 10 angeordnet und in einem Schritt 102 die Vergrößerung des Zoomsystems 16 auf den Wert Γ = 2,4 eingestellt. In einem Schritt 104 wird das Operationsmikroskop 10 dann auf das Muster 62 fokussiert. In dem Schritt 106 wird anschließend mit der Anzeigeeinrichtung 34 ein auf den zu dem Muster 62 in der Rechnereinheit 42 berechneten Bilddaten basierendes Bild 82 erzeugt. Dieses Bild wird dem in der Fig. 4 gezeigten Bildmuster 88 in dem Binokulartubus 18 des Operationsmikroskops 10 in Überlagerung zu dem Beobachtungsbild des Objektbereichs 20, in dem sich das in der Fig. 3 abgebildeten Referenzobjekt 60 mit dem Muster 62 befinden, zur Anzeige gebracht. In einem Schritt 108 wird das Bildmuster 88 dann mit dem Muster 62 verglichen und durch Einstellen wenigstens eines Visualisierungsparameters für die mit der Anzeigeeinrichtung 34 angezeigte Orientierungsinformation in Form der Position und/oder der azimutalen Orientierung und/oder der Vergrößerung des Bildes 86 das Muster 62 mit dem Bildmuster 88 verlagert und damit wenigstens teilweise zur Deckung gebracht. Dabei entspricht der zu einer Vergrößerung des Operationsmikroskops 10 ermittelte Korrekturwert für die Position, die azimutale Orientierung und die Vergrößerung des Bildes 86 den gesuchten systematischen Fehlern für die an dem Display 36 der Anzeigeeinrichtung 34 in dem Operationsmikroskop 10 zur Anzeige gebrachten Bilder mit einer Orientierungsinformation. In einem Schritt 110 werden diese systematischen Fehler dann abgespeichert. Daran anschließend werden für die Vergrößerungen Γ = 0,4 und Γ = 1,0 die Schritte 106, 108 und 110 entsprechend durchgeführt, um die systematischen Fehler für die an dem Display 36 zur Anzeige gebrachten Bilder mit Orientierungsinformation zu ermitteln und in dem Datenspeicher 52 des Rechnersystems 43 abzulegen.

Es sei bemerkt, dass in einer zu dem vorstehend beschriebenen Ausführungsbeispiel modifizierten Ausführung der Erfindung die Visualisierungsvorrichtung insbesondere auch als Digiskopie-Vorrichtung oder als Video-Mikroskop, insbesondere als Video-Operationsmikroskop ausgebildet sein kann, d.h. als Visualisierungsvorrichtung, die keinen durchgängigen optischen Strahlengang hat, mit dem das Bild des Objektbereichs den Augen einer Beobachtungsperson ausgehend vom Objektbereich durch ein Okular zur Anzeige gebracht wird. In einer solchen Visualisierungsvorrichtung hat die Anzeigeeinrichtung eine Doppelfunktion. Sie dient sowohl für das Visualisieren des Beobachtungsbildes des Objektbereichs als auch für das Visualisieren eines dem Beobachtungsbild überlagerten Bildes, das Orientierungsinformation enthält.

Zusammenfassend ist insbesondere folgendes festzuhalten: Die Erfindung betrifft eine Visualisierungsvorrichtung 10, die eine Abbildungsoptik 12 für das Erzeugen eines Beobachtungsbildes eines Objektbereichs 20 enthält und eine Anzeigeeinrichtung 34 mit einem Display 36 für das Visualisieren eines dem Beobachtungsbild des Objektbereichs 20 überlagerten Bildes 54 mit einer Orientierungsinformation aufweist. Die Visualisierungsvorrichtung hat eine Bilderfassungseinrichtung 26 für das Erfassen eines Bilds des Objektbereichs. Die Visualisierungsvorrichtung 10 hat ein Rechnersystem 43, das aus einem mit der Bilderfassungseinrichtung 26 erfassten Bild des Objektbereichs die Orientierungsinformation enthaltenden Bilddaten berechnet und an die Anzeigeeinrichtung 34 übermittelt. Die Abbildungsoptik umfasst dabei einen Binokulartubus 18 für das Visualisieren des Objektsbereichs mit einem optischen Beobachtungsstrahlengang 22a, 22b und einen in dem optischen Beobachtungsstrahlengang 22a, 22b angeordneten Strahlteiler 30, der das Beobachtungsbild des Objektsbereichs 20 der Bilderfassungseinrichtung 26 zuführt. Die Abbildungsoptik weist auch einen in dem optischen Strahlengang 22a, 22b angeordneten Strahlteiler 40 auf, der das mit der Anzeigeeinrichtung 34 angezeigte Bild 46 dem Beobachtungsbild des Objektbereichs 20 überlagert und dem Binokulartubus 18 zuführt. Das Rechnersystem 43 korrigiert bei der Berechnung der Bilddaten wenigstens einen von Fertigungs-, Einbau-, sowie Justagetoleranzen des Displays 36 hervorgerufenen systematischen Fehler des mit der Anzeigeeinrichtung 34 angezeigten Bildes 46 gegenüber dem Beobachtungsbild, indem es für das dem Beobachtungsbild überlagerte, angezeigte Bild 54 wenigstens einen Korrekturparameter aus der Parametergruppe Vergrößerung, azimutale Verdrehung und Verschiebung einstellt, der aus einem Vergleich des Beobachtungsbildes eines Referenzobjekts 60 mit einem dem Beobachtungsbild überlagerten und mit der Anzeigeneinrichtung 34 visualisierten Vergleichsobjekt ermittelt ist.

### Bezugszeichenliste:

- 10: Operationsmikroskop
- 12: Abbildungsoptik
- 14: Mikroskop-Hauptobjektiv
- 16: Zoomsystem
- 18: Binokulartubus
- 19: Zwischenbild
- 20: Objektbereich
- 22a, 22b: Beobachtungsstrahlengang
- 24: Augen
- 26: Bilderfassungseinrichtung
- 28: Bildsensor
- 30: Strahlteiler
- 32: Bilderfassungsoptik
- 34: Anzeigeeinrichtung
- 36: Display
- 38: Abbildungsoptik
- 40: Strahlteiler
- 41: geometrische Struktur
- 42: Rechnereinheit
- 43: Rechnersystem
- 44: Eingabeschnittstelle
- 45: Monitor
- 46, 54: Bild
- 48: Pfeil
- 49, 49': Orientierungsinformation, Hilfsgeometrie
- 50: Zentrum
- 52: Datenspeicher
- 55: Parameterkennfeld
- 60: Referenzobjekt
- 62: Muster
- 64: Zentrum
- 66: Kreisscheibe
- 68: Kreisring
- 70,72: Markierungen
- 74,76: Linien
- 78, 80, 82, 84: Kreislinien
- 86: Bild
- 87: Azimutwinkelmarkierung
- 87a, 87b, 87c: Winkelstriche
- 88: Bildmuster
- 90: Fadenkreuz
- 92: Zentrum
- 94,96: Kreisringe
- 100: Flussdiagramm
- 102,104,106, 108, 110: Schritt

## Patentansprüche

1. Visualisierungsvorrichtung (10),
mit einer Abbildungsoptik (12) für das Erzeugen eines Beobachtungsbildes eines Objektbereichs (20);
mit einer ein Display (36) enthaltenden Anzeigeeinrichtung (34) für das Visualisieren eines dem Beobachtungsbild des Objektbereichs (20) überlagerten Bildes (54) mit einer Orientierungsinformation;
mit einer Bilderfassungseinrichtung (26) für das Erfassen eines Bilds des Objektbereichs; und
mit einem Rechnersystem (43), das aus einem mit der Bilderfassungseinrichtung (26) erfassten Bild des Objektbereichs die Orientierungsinformation enthaltenden Bilddaten berechnet und an die Anzeigeeinrichtung (34) übermittelt; wobei
die Abbildungsoptik einen Binokulartubus (18) für das Visualisieren des Objektsbereichs mit einem optischen Beobachtungsstrahlengang (22a, 22b) und einen in dem optischen Beobachtungsstrahlengang (22a, 22b) angeordneten Strahlteiler (30) umfasst, der das Beobachtungsbild des Objektsbereichs (20) der Bilderfassungseinrichtung (26) zuführt und einen in dem optischen Strahlengang (22a, 22b) angeordneten Strahlteiler (40) aufweist, der das mit der Anzeigeeinrichtung (34) angezeigte Bild (46) dem Beobachtungsbild des Objektbereichs (20) überlagert und dem Binokulartubus (18) zuführt, und wobei
das Rechnersystem (43) bei der Berechnung der Bilddaten wenigstens einen von Fertigungs-, Einbau-, sowie Justagetoleranzen des Displays (36) hervorgerufenen systematischen Fehler des mit der Anzeigeeinrichtung (34) angezeigten Bildes (46) gegenüber dem Beobachtungsbild korrigiert, indem es für das dem Beobachtungsbild überlagerte, angezeigte Bild (54) wenigstens einen Korrekturparameter aus der Parametergruppe Vergrößerung, azimutale Verdrehung und Verschiebung einstellt, der aus einem Vergleich des Beobachtungsbildes eines Referenzobjekts (60) mit einem dem Beobachtungsbild überlagerten und mit der Anzeigeneinrichtung (34) visualisierten Vergleichsobjekt ermittelt ist,
**dadurch gekennzeichnet, dass**
die Abbildungsoptik (12) ein Vergrößerungssystem (16) mit einer einstellbaren Vergrößerung enthält und das Rechnersystem (43) eine Eingabeschnittstelle (44) für das Eingeben des wenigstens einen Korrekturparameters und für das Eingeben eines Anzeigeparameters zu unterschiedlichen Vergrößerungen sowie einen Datenspeicher (52) aufweist, der den an der Eingabeschnittstelle (44) zu unterschiedlichen Vergrößerungen eingegebenen Anzeigeparameter für das mit der Anzeigeeinrichtung (34) angezeigte Bild speichert, wobei das Rechnersystem (43) bei der Berechnung der für die Anzeige des Bildes (46) an die Anzeigeeinrichtung (34) übermittelten Bilddaten die Vergrößerung des Vergrößerungssystems (16) und die zu unterschiedlichen Vergrößerungen eingegebenen Korrekturparameter berücksichtigt.

2. Visualisierungsvorrichtung nach Anspruch 1, **dadurch gekennzeichnet, dass** das Rechnersystem (43) mit dem Vergrößerungssystem (16) verbunden ist und eine Rechnereinheit (42) umfasst, die den wenigstens einen Korrekturparameter durch Auslesen und/oder Extrapolieren eines in dem Datenspeicher (52) abgelegten Parameterkennfelds (54) mit zu unterschiedlichen Vergrößerungen des Vergrößerungssystems (16) hinterlegten Korrekturparametern bestimmt.

3. Als Operationsmikroskop ausgebildete Visualisierungsvorrichtung nach Anspruch 1 oder 2.

4. Verfahren für das Ermitteln eines systematischen Fehlers eines dem Beobachtungsbild eines Objektbereichs (20) in
einer gemäß Anspruch 1 oder 2 ausgebildeten Visualisierungsvorrichtung (10)
oder in einem Operationsmikroskop gemäß Anspruch 3 überlagerten, mit der Anzeigeeinrichtung (34) angezeigten Bildes (54),
**gekennzeichnet durch** folgende Schritte:
Bereitstellen des Referenzobjekts (60) in dem Objektbereich (20) mit einer wenigstens die Lage und eine Länge sowie eine azimutale Orientierung des Referenzobjekts (60) definierenden geometrischen Struktur 74, 76, 78);
wenigstens teilweises Anpassen einer geometrischen Struktur (90, 94, 96) eines Bildmusters (88) in dem berechneten Bild (86) an die geometrische Struktur (74, 76, 78) des in dem Beobachtungsbildes angezeigten Referenzobjekts (60) durch Einstellen wenigstens eines Korrekturparameters für das mit der Anzeigeeinrichtung (34) angezeigte Bild (86) aus der Parametergruppe Vergrößerung, azimutale Verdrehung und Verschiebung; und
Bestimmen des systematischen Fehlers als denjenigen wenigstens einen eingestellten Korrekturparameter, bei dem wenigstens eine geometrische Struktur (94, 96) des Bildes (86) an eine dieser Struktur (94, 96) entsprechende geometrische Struktur (68) des angezeigten Beobachtungsbildes des Referenzobjekts (60) wenigstens teilweise angepasst ist, wobei
der systematische Fehler für unterschiedliche Vergrößerungen der Visualisierungsvorrichtung (10) ermittelt wird.

5. Verwendung eines Referenzobjekts (60) mit einem die Lage (64) und eine Länge (78, 80, 82, 84) und die azimutale Orientierung (74, 76) definierenden Strukturen aufweisenden Musters (62) für das Justieren der Anzeige eines mit der Anzeigeeinrichtung (34) in einer gemäß Anspruch 1 oder Anspruch 2 ausgebildeten Visualisierungsvorrichtung oder in einem Operationsmikroskop gemäß Anspruch 3 angezeigten Bildes.

6. Verfahren für das Anpassen eines dem Beobachtungsbild eines Objektbereichs (20) in einer gemäß Anspruch 1 oder Anspruch 2 ausgebildeten Visualisierungsvorrichtung oder in einem Operationsmikroskop gemäß Anspruch 3 überlagerten angezeigten berechneten Bildes (86), **dadurch gekennzeichnet, dass** ein mit einem Verfahren gemäß Anspruch 4 ermittelter systematischer Fehler des dem Beobachtungsbild des Objektbereichs (20) überlagerten mit der Anzeigeeinrichtung (34) angezeigten Bildes (54) durch Einstellen wenigstens eines Korrekturparameters für das mit der Anzeigeeinrichtung (34) angezeigte Bild (46) aus der Parametergruppe Vergrößerung, azimutale Verdrehung und Verschiebung kompensiert wird.

7. Verfahren zum Ausgleichen eines systematischen Fehlers von mit der Anzeigeeinrichtung (34) in einer gemäß Anspruch 1 oder Anspruch 2 ausgebildeten Visualisierungsvorrichtung (10) oder in einem gemäß Anspruch 3 ausgebildeten Operationsmikroskop angezeigten Orientierungsinformation, **dadurch gekennzeichnet, dass** aus einem Bild eines Objektbereichs (20), das einem Bildsensor (28) zugeführt wird, Bilddaten für eine Anzeigeeinrichtung (34) berechnet werden und bei der Berechnung der an die Anzeigeeinrichtung (34) übermittelten Bilddaten wenigstens ein der Rechnereinheit (42) zuführbarer Korrekturparameter aus der Parametergruppe Vergrößerung, azimutale Verdrehung und Verschiebung berücksichtigt wird.

8. Computerprogramm zur Durchführung des Verfahrens nach Anspruch 7.

9. Computerprogramm nach Anspruch 8, **dadurch gekennzeichnet, dass** es bei der Berechnung der für die Anzeige des Bildes (46) an die Anzeigeeinrichtung (34) übermittelten Bilddaten die Vergrößerung des Beobachtungsbildes und zu unterschiedlichen Vergrößerungen des Beobachtungsbildes ermittelte Korrekturparameter berücksichtigt.

10. Computerprogramm nach Anspruch 8 oder 9, **dadurch gekennzeichnet, dass** es das dem Bildsensor (28) zugeführte Bild des Objektbereichs (20) mit einer Vergleichsinformation in Form von über das erfasste Bild des Objektbereichs gelegten Vergleichsobjekten in Form von Ringfiltern, die einen inneren und einen äußeren Filterring haben, und in Form von Kreisringen mit wenigstens einer in einem Kreisring angeordneten Markierung korreliert.

## Claims

1. Visualization apparatus (10),
having an imaging optical unit (12) for producing an observation image of an object region (20);
having a display device (34) containing a display (36) for visualizing an image (54) having a piece of orientation information, which is superposed on the observation image of the object region (20);
having an image capturing device (26) for capturing an image of the object region; and
having a computational system (43) that calculates image data containing the orientation information from an image of the object region that was captured with the image capturing device (26) and transmits them to the display device (34); wherein
the imaging optical unit comprises a binocular tube (18) for visualizing the object region with an optical observation beam path (22a, 22b) and a beam splitter (30) that is arranged in the optical observation beam path (22a, 22b), said beam splitter (30) feeding the observation image of the object region (20) to the image capturing device (26), and has a beam splitter (40) that is arranged in the optical beam path (22a, 22b) and superposes the image (46) displayed with the display device (34) on the observation image of the object region (20) and feeds it to the binocular tube (18), and wherein the computational system (43) corrects with respect to the observation image at least one systematic error, caused by manufacturing, installation and adjustment tolerances of the display (36), of the image (46) displayed with the display device (34) during the calculation of the image data by way of setting, for the displayed image (54) that is superposed on the observation image, at least one correction parameter from the parameter group magnification, azimuthal rotation and displacement that is ascertained from a comparison of the observation image of a reference object (60) to a comparison object that is superposed on the observation image and visualized with the display device (34),
**characterized in that**
the imaging optical unit (12) contains a magnification system (16) having a settable magnification and the computational system (43) has an input interface (44) for inputting the at least one correction parameter and for inputting a display parameter for different magnifications, and a data memory (52) that stores the display parameters that are input for different magnifications at the input interface (44) for the image that is displayed with the display device (34), wherein the computational system (43) takes the magnification of the magnification system (16) and the correction parameters input for different magnifications into consideration when calculating the image data that are transmitted to the display device (34) for the display of the image (46).

2. Visualization apparatus according to Claim 1, **characterized in that** the computational system (43) is connected to the magnification system (16) and comprises a computational unit (42) that determines the at least one correction parameter by reading and/or extrapolating a parameter characteristic map (54), stored in the data memory (52), with correction parameters which are stored for different magnifications of the magnification system (16) .

3. Visualization apparatus, embodied in the form of a surgical microscope, according to Claim 1 or 2.

4. Method for ascertaining a systematic error of an image (54) that is displayed with the display device (34) and that is superposed on the observation image of an object region (20) in
a visualization apparatus (10) embodied according to Claim 1 or 2
or in a surgical microscope according to Claim 3, **characterized by** the following steps:
providing the reference object (60) in the object region (20) with a geometric structure (74, 76, 78) that defines at least the position and a length and an azimuthal orientation of the reference object (60);
at least partially adapting a geometric structure (90, 94, 96) of an image pattern (88) in the calculated image (86) to the geometric structure (74, 76, 78) of the reference object (60) displayed in the observation image by setting at least one correction parameter from the parameter group magnification, azimuthal rotation and displacement for the image (86) displayed with the display device (34); and
determining the systematic error as the at least one set correction parameter in which at least one geometric structure (94, 96) of the image (86) is at least partially adapted to a geometric structure (68) of the displayed observation image of the reference object (60) that corresponds to said structure (94, 96), wherein
the systematic error is ascertained for different magnifications of the visualization apparatus (10).

5. Use of a reference object (60) with a pattern (62) that has structures which define the position (64) and a length (78, 80, 82, 84) and the azimuthal orientation (74, 76) for adjusting the display of an image that is displayed with the display device (34) in a visualization apparatus embodied according to Claim 1 or Claim 2 or in a surgical microscope according to Claim 3.

6. Method for adapting a displayed calculated image (86) that is superposed on the observation image of an object region (20) in a visualization apparatus embodied according to Claim 1 or Claim 2 or in a surgical microscope according to Claim 3, **characterized in that** a systematic error, ascertained with a method according to Claim 4, of the image (54), which is superposed on the observation image of the object region (20) and displayed with the display device (34), is compensated by setting at least one correction parameter from the parameter group magnification, azimuthal rotation and displacement for the image (46) displayed with the display device (34) .

7. Method for compensating a systematic error of orientation information displayed with the display device (34) in a visualization apparatus (10) embodied according to Claim 1 or Claim 2 or in a surgical microscope embodied according to Claim 3, **characterized in that** image data are calculated for a display device (34) from an image of an object region (20) that is fed to an image sensor (28) and at least one correction parameter from the parameter group magnification, azimuthal rotation and displacement that is able to be fed to the computational unit (42) is taken into account in the calculation of the image data transmitted to the display device (34).

8. Computer program for performing the method according to Claim 7.

9. Computer program according to Claim 8, **characterized in that** it takes into account the magnification of the observation image and correction parameters ascertained for different magnifications of the observation image when calculating the image data transmitted to the display device (34) for the display of the image (46).

10. Computer program according to Claim 8 or 9, **characterized in that** it correlates the image of the object region (20) supplied to the image sensor (28) to a piece of comparison information in the form of comparison objects, placed over the captured image of the object region, in the form of ring filters that have an inner and an outer filter ring and in the form of circular rings having at least one mark arranged in a circular ring.

## Revendications

1. Dispositif de visualisation (10),
pourvu d'une optique de reproduction (12) destinée à générer une image d'observation d'une zone d'objet (20) ;
pourvu d'un système d'affichage (34) contenant un écran (36) destiné à la visualisation d'une image (54) superposée à l'image d'observation de la zone d'objet (20) avec une information d'orientation ;
pourvu d'un système de capture d'image (26) destiné à capturer une image de la zone d'objet ; et
pourvu d'un système informatique (43), qui à partir d'une image de la zone d'objet capturée à l'aide du système de capture d'image (26) calcule des données d'image contenant l'information d'orientation et les transfère au système d'affichage (34) ;
l'optique de reproduction comprenant un tube binoculaire (18) destiné à la visualisation de la zone d'objet à l'aide d'une trajectoire de faisceau optique d'observation (22a, 22b) et un séparateur de faisceau (30) placé dans la trajectoire de faisceau optique d'observation (22a, 22b), qui amène l'image d'observation de la zone d'objet (20) au système de capture d'image (26) et qui comporte un séparateur de faisceau (40) placé dans la trajectoire de faisceau optique (22a, 22b) qui superpose l'image (46) affichée à l'aide du système d'affichage (34) à l'image d'observation de la zone d'objet (20) et l'amène au tube binoculaire (18), et
lors du calcul des données d'image, le système informatique (43) corrigeant au moins un défaut systématique de l'image (46) affichée à l'aide du système d'affichage (34) qui est dû à des tolérances de fabrication, de montage et d'ajustage de l'écran (36) par rapport à l'image d'observation en ce que pour l'image (54) affichée, superposée à l'image d'observation, il règle au moins un paramètre de correction relevant du groupe de paramètres agrandissement, rotation azimutale et décalage, qui est déterminé par une comparaison de l'image d'observation d'un objet de référence (60) avec un objet témoin superposé à l'image d'observation et visualisé à l'aide du système d'affichage (34), **caractérisé en ce que** l'optique de reproduction (12) contient un système d'agrandissement (16) avec un agrandissement réglable et le système informatique (43) comporte une interface de saisie (44), destinée à saisir l'au moins un paramètre de correction et à saisir un paramètre d'affichage pour différents agrandissements, ainsi qu'une mémoire de données (52) qui mémorise le paramètre d'affichage saisi sur l'interface de saisie (44) pour différents agrandissements pour l'image affichée à l'aide du système d'affichage (34), lors du calcul des données d'image transférées pour l'affichage de l'image (46) au système d'affichage (34), le système informatique (43) tenant compte de l'agrandissement du système d'agrandissement (16) et des paramètres de correction saisis pour différents agrandissements.

2. Dispositif de visualisation selon la revendication 1, **caractérisé en ce que** le système informatique (43) est connecté avec le système d'agrandissement (16) et comprend une unité informatique (42) qui détermine l'au moins un paramètre de correction par lecture et/ou par extrapolation d'un diagramme caractéristique de paramètres (54) sauvegardé dans la mémoire de données (52), comprenant des paramètres de correction sauvegardés pour différents agrandissements du système d'agrandissement (16).

3. Dispositif de visualisation selon la revendication 1 ou 2, conçu sous la forme d'un microscope chirurgical.

4. Procédé, destiné à déterminer un défaut systématique d'une image (54) qui est affichée à l'aide du système d'affichage (34) et qui est superposée à l'image d'observation d'une zone d'objet (20) dans un dispositif de visualisation (10) conçu selon la revendication 1 ou 2 ou
dans un microscope chirurgical selon la revendication 3, **caractérisé par** les étapes suivantes, consistant à :
mettre à disposition l'objet de référence (60) dans la zone d'objet (20) avec une structure géométrique (74, 76, 78) définissant au moins la position et une longueur, ainsi qu'une orientation azimutale de l'objet de référence (60) ;
adapter au moins partiellement une structure géométrique (90, 94, 96) d'un motif d'image (88) dans l'image (86) calculée à la structure géométrique (74, 76, 78) de l'objet de référence (60) affiché dans l'image d'observation, par réglage d'au moins paramètre de correction pour l'image (86) affichée à l'aide du système d'affichage (34) du groupe de paramètres agrandissement, rotation azimutale et décalage ; et
déterminer le défaut systématique comme celui d'au moins un paramètre de correction réglé, avec lequel au moins une structure géométrique (94, 96) de l'image (86) est adaptée au moins partiellement à une structure géométrique (68), correspondant à ladite structure (94, 96), de l'image d'observation affichée de l'objet de référence (60),
le défaut systématique étant déterminé pour différents agrandissements du dispositif de visualisation (10).

5. Utilisation d'un objet de référence (60) avec un motif (62) comportant des structures définissant la position (64) et une longueur (78, 80, 82, 84) et l'orientation azimutale (74, 76), pour ajuster l'affichage d'une image qui est affichée avec le système d'affichage (34) dans un dispositif de visualisation conçu selon la revendication 1 ou la revendication 2 ou dans un microscope chirurgical selon la revendication 3.

6. Procédé, destiné à adapter une image (86) calculée affichée en étant superposée à l'image d'observation d'une zone d'objet (20) dans un dispositif de visualisation conçu selon la revendication 1 ou la revendication 2 ou dans un microscope chirurgical selon la revendication 3, **caractérisé en ce qu'**on compense un défaut systématique déterminé à l'aide d'un procédé selon la revendication 4 de l'image (54) superposée à l'image d'observation de la zone d'objet (20), affichée à l'aide du système d'affichage (34) par réglage d'au moins un paramètre de correction pour l'image (46) affichée à l'aide du système d'affichage (34) du groupe de paramètres agrandissement, rotation azimutale et décalage.

7. Procédé, destiné à compenser un défaut systématique de l'information d'orientation affichée à l'aide du système d'affichage (34) dans un dispositif de visualisation conçu (10) selon la revendication 1 ou la revendication 2 ou dans un microscope chirurgical conçu selon la revendication 3, **caractérisé en ce qu'**à partir d'une image d'une zone d'objet (20), qui est amenée à un capteur d'image (28), on calcule des données d'image pour un système d'affichage (34) et lors du calcul des données d'image transférées au système d'affichage (34), on tient compte d'au moins un paramètre de correction susceptible d'être amené à l'unité informatique (42) du groupe de paramètres agrandissement, rotation azimutale et décalage.

8. Programme informatique, destiné à réaliser le procédé selon la revendication 7.

9. Programme informatique selon la revendication 8, **caractérisé en ce que** lors du calcul des données d'image transférées au système d'affichage (34) pour l'affichage de l'image (46), il prend en compte l'agrandissement de l'image d'observation et des paramètres de correction déterminés pour différents agrandissements de l'image d'observation.

10. Programme informatique selon la revendication 8 ou 9, **caractérisé en ce qu'**il met en corrélation l'image de la zone d'objet (20) amenée au capteur d'image (28) avec une information comparative sous la forme d'objets témoins posés au-dessus de l'image capturée de la zone d'objet sous la forme de filtres annulaires disposant d'un anneau de filtrage intérieur et d'un anneau de filtrage extérieur et sous la forme de cercles annulaires pourvus d'au moins un marquage placé dans un cercle annulaire.
